# EUROPÄISCHE PATENTSCHRIFT

(11) **EP 2 226 319 B1**
(45) Veröffentlichungstag und Bekanntmachung des Hinweises auf die Patenterteilung: **16.03.2016**
(21) Anmeldenummer: 10163868.2
(22) Anmeldetag: 17.05.2006
(51) Int. Cl.: C07D 403/04

(54) **Verfahren zur Herstellung von 2-(2-Aminopyrimidin-4-yl)-1H-indol-5-carbonsäurederivaten**
Process for the preparation of 2-(2-aminopyrimidin-4-yl)-1H-indol-5-carbonic acid derivatives
Procédé de préparation de dérivés d'acide carbonique 2-(2-aminopyrimidin-4-yl)-1H-indole-5

(30) Priorität: 01.06.2005 DE 102005025225
(43) Veröffentlichungstag der Anmeldung: 08.09.2010
(62) Teilanmeldung aus: 06753669.8
(73) Patentinhaber: SANOFI, 75008 Paris (FR)
(72) Erfinder: Dr. Graeser, Joachim, 65926 Frankfurt am Main (DE); Dr. Billen, Guenter, 65926 Frankfurt am Main (DE); Dr. Linkies, Adolf, 65926 Frankfurt am Main (DE); Dr. Metzenthin, Tobias, 65926 Frankfurt am Main (DE)
(74) Vertreter: Then, Johann

(56) Entgegenhaltungen:
- WO-A-2004/022553
- DATABASE CA [Online] CHEMICAL ABSTRACTS SERVICE, COLUMBUS, OHIO, US; 2000, XP002397851 gefunden im STN Database accession no. 2000:499651 & JIANG, BIAO ET AL: "Synthesis of indolylpyrimidines via cross-coupling of indolylboronic acid with chloropyrimidines: facile synthesis of meridianin D" HETEROCYCLES , CODEN: HTCYAM; ISSN: 0385-5414, Bd. 53, Nr. 7, 2000, Seiten 1489-1498,

## Beschreibung

Die Erfindung betrifft ein Verfahren zur Herstellung von Verbindungen der Formel I und neue Zwischenprodukte bei der Gewinnung der Verbindung der Formel I. Die genannten Indolderivate sind geeignete Zwischenprodukte zur Herstellung von IkB-Kinase Inhibitoren (WO 01/30774 A1; WO2004/022553).

Es ist bekannt, dass man Indolderivate als Bausteine für die Synthese von pharmazeutischen Wirkstoffen einsetzt. Beispielsweise sind 2-(2-Amino-pyrimidin-4-yl)-1H-indol-5-carbonsäuren oder deren Salze wichtige Bausteine für die Herstellung von IkB-Kinase Inhibitoren (siehe WO 01/30774 A1):

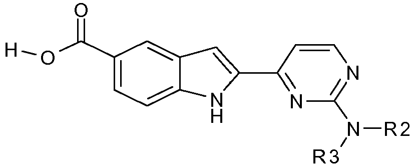

2-(2-Amino-pyrimidin-4-yl)-1H-indol-5-carbonsäuren können durch klassische Fischer-Indolsynthese ausgehend von den entsprechenden 4-Acetylpyrimidinen (III) und 4-Hydrazinobenzoesäure (II) hergestellt werden (siehe Schema 1):

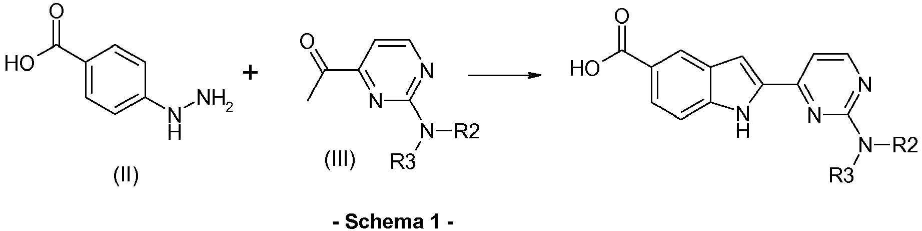

Nachteilig sind hierbei zum einen die drastischen Reaktionsbedingungen, die für eine vollständige Umsetzung benötigt werden. Zum anderen fallen die Produkte dieser Reaktion im Gemisch mit den entsprechenden Oligomeren an, was zu einer schlechten Isolierbarkeit führt, insbesondere in Hinblick auf die Filtrationszeit. Ferner lassen sich diese Oligomere aufgrund der geringen Löslichkeit von 2-(2-Amino-pyrimidin-4-yl)-1H-indol-5-carbonsäuren in organischen Lösungsmitteln nur schlecht abtrennen und werden bei den weiteren Reaktionen teilweise bis zum Wirkstoff als Verunreinigung mitgeschleppt.

Aufgabe der vorliegenden Erfindung ist es, ein technisches Verfahren zur Herstellung der Verbindungen der Formel I zu finden, welche die genannten Nachteile nicht aufweist.

Es wurde nun ein Verfahren zur Gewinnung der Verbindung der Formel I gefunden, 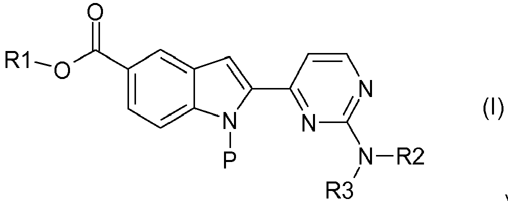 wobei
R1 für
   1) Wasserstoffatom,
   2) -(C₁-C₁₂)-Alkyl,
   3) -(C₆-C₁₄)-Aryl,
   4) -(C₃-C₈)-Cycloalkyl oder
   5) 4- bis 15-gliedriger Het-Ring, steht,
R2 und R3 gleich oder verschieden sind und unabhängig voneinander für
   1) Wasserstoffatom,
   2) -(C₁-C₁₂)-Alkyl,
   3) -(C₆-C₁₄)-Aryl, wobei Aryl unsubstituiert oder ein, zwei oder dreifach durch
      -(C₁-C₆)-Alkyl substituiert ist,
   4) -(C₃-C₈)-Cycloalkyl oder
   5) 4- bis 15-gliedriger Het-Ring, stehen,
P für Wasserstoffatom oder für eine N-Schutzgruppe steht,
   das dadurch gekennzeichnet ist, dass man
   a) ein Boronoindol der Formel IV 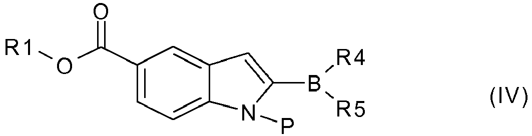
      worin R1 die gleiche Bedeutung wie in Formel I hat,
      R4 und R5 gleich oder verschieden sind und unabhängig voneinander für
         1) -OH,
         2) -O-(C₁-C₁₂)-Alkyl,
         3) -O-(C₆-C₁₄)-Aryl,
         4) -O-(C₃-C₈)-Cycloalkyl,
         5) -(C₁-C₁₂)-Alkyl oder
         6) -O-Het, wobei Het ein 4- bis 15-gliedriger Het-Ring bedeutet, stehen, oder
      R4 und R5 bilden zusammen mit dem B-Atom an das sie gebunden sind einen Ring mit 4, 5, 6 oder 7 Kohlenstoffatomen im Ring und der Ring kann statt der jeweiligen Kohlenstoffatome zwei Sauerstoffatome oder zwei Sauerstoffatome und ein Stickstoffatom enthalten,
      P für Wasserstoffatom oder für eine N-Schutzgruppe steht, mit einem Aminopyrimidin der Formel V umsetzt, 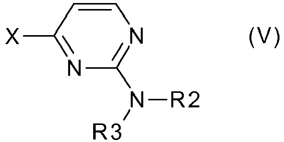 worin R2 und R3 die gleiche Bedeutung wie in Formel I haben und
         X für
            1) Halogen,
            2) -O-SO₂-R2, oder
            3) -O-C(O)-R2 steht,
            und die gegebenenfalls vorhandene N-Schutzgruppe abspaltet, oder
   b) ein Boronoindol der Formel IV mit einem Pyrimidin der Formel VI, 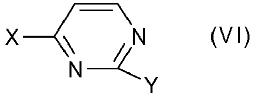
      worin X die gleiche Bedeutung wie in Formel V hat und
      Y für
         1) Halogen,
         2) -O-SO₂-R2, oder
         3) -O-C(O)-R2 steht,
         zu einer Verbindung der Formel VII umsetzt, 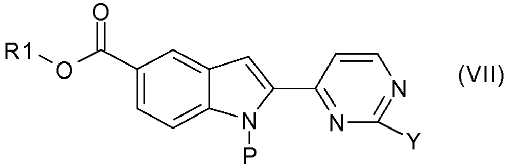 worin R1 und P die Bedeutung in der Verbindung der Formel IV haben und Y die gleiche Bedeutung wie in der Verbindung der Formel VI hat, und anschließend die Verbindung der Formel VII mit einem Amin der Formel VIII 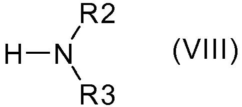 worin R2 und R3 die gleiche Bedeutung wie in Formel I haben, zu einer Verbindung der Formel I umsetzt und die gegebenenfalls vorhandene
      N-Schutzgruppe abspaltet, oder
   c) ein Alkin der Formel IX, 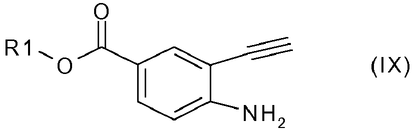 worin R1 die gleiche Bedeutung wie in Formel I hat,
      mit einem Pyrimidin der Formel (V) zu einem Alkin der Formel X umsetzt, 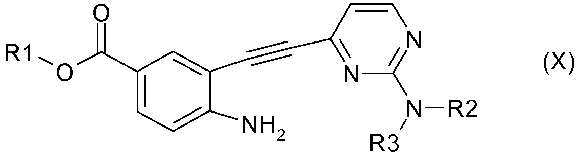 worin R1, R2 und R3 die gleiche Bedeutung wie in Formel I haben, und die Verbindung der Formel X durch Ringschluss in die Verbindung der Formel I überführt wird oder
   d) die nach den Verfahren a), b) oder c) hergestellte Verbindung der Formel I entweder in freier Form isoliert oder im Falle des Vorliegens von sauren oder basischen Gruppen in physiologisch verträgliche Salze umwandelt.

Die Erfindung betrifft ferner ein Verfahren zur Gewinnung der Verbindung der Formel I, wobei
R1 für
1) Wasserstoffatom,
2) -(C₁-C₆)-Alkyl,
3) Phenyl oder
4) -(C₃-C₆)-Cycloalkyl, steht,
R2 und R3 gleich oder verschieden sind und unabhängig voneinander für Wasserstoffatom oder
-(C₁-C₄)-Alkyl, stehen,
das dadurch gekennzeichnet ist, dass man ein Boronoindol der Formel IV, worin R1 wie für Formel I definiert ist,
R4 und R5 gleich oder verschieden sind und unabhängig voneinander für -OH oder
-(C₁-C₆)-Alkyl, stehen, oder
R4 und R5 bilden zusammen mit dem B-Atom an das sie gebunden sind einen Ring aus der Reihe Borolan, Borinan, Borepan, Borocan, [1,3,2]Dioxaborolan, [1,3,2]Dioxaborinan, [1,3,2]Dioxaborepan, [1,3,2]Dioxaborocan oder [1,3,6,2]Dioxazaborocan,
P für
1) Wasserstoffatom,
2) -C(O)-O-R6, worin R6 für
   a) Wasserstoffatom,
   b) -(C₁-C₆)-Alkyl,
   c) -(C₆-C₁₄)-Aryl, wobei Aryl ausgewählt ist aus der Reihe Phenyl, Naphthyl, Anthryl oder Fluorenyl und worin Aryl unsubstituiert oder ein, zwei oder dreifach durch -(C₁-C₆)-Alkyl substituiert ist,
   d) -(C₃-C₆)-Cycloalkyl oder
   e) für einen Rest aus der Reihe Acridinyl, Azepinyl, Azetidinyl, Aziridinyl, Benzimidazalinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4αH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxodiazolyl, 1,2,4-Oxadiazolyl, 1,2,5-Oxodiazolyl, 1,3,4-Oxadiazolyl, Oxazolidinyl, Oxazolyl, Oxazolidinyl, Oxothiolanyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Thiomorpholinyl, Thiophenyl, Triazinyl, 1,2,3-Triazolyl, 1,2,3-Triazolyl, 1,2,4-Triazolyl, 1,2,5-Triazolyl, 1,3,4-Triazolyl oder Xanthenyl, steht,
3) -SO₃-R6,
4) -O-SO₂-R6,
5) -Si-R6 oder
6) Benzyl steht,
mit einem Aminopyrimidin der Formel V umsetzt,
worin R2 und R3 gleich oder verschieden sind und unabhängig voneinander für Wasserstoffatom oder -(C₁-C₄)-Alkyl stehen, und
X für Cl, Br, I, -O-Tosylat, -O-Mesylat oder-O-Acetat steht, und
die gegebenenfalls vorhandene N-Schutzgruppe abspaltet.

Die Erfindung betrifft ferner ein Verfahren zur Gewinnung der Verbindung der Formel I wobei
R1 für Wasserstoffatom oder Ethyl steht,
R2 und R3 gleich oder verschieden sind und unabhängig voneinander für Wasserstoffatom oder
-(C₁-C₄)-Alkyl, stehen, das dadurch gekennzeichnet ist, dass man ein Boronoindol der Formel IV, worin R1 wie für Formel I definiert ist,
R4 und R5 gleich sind und für-OH stehen, und
P für
1) Wasserstoffatom,
2) -O-Tosylat oder
3) Benzyl steht,
mit einem Aminopyrimidin der Formel V umsetzt,
worin R2 und R3 gleich oder verschieden sind und unabhängig voneinander für Wasserstoffatom oder -(C₁-C4)-Alkyl stehen, und
X für Cl, Br, I, -O-Tosylat, -O-Mesylat oder-O-Acetat steht und
die gegebenenfalls vorhandene N-Schutzgruppe abspaltet.

Die Erfindung betrifft ferner ein Verfahren zur Gewinnung der Verbindung der Formel I wobei
R1 für
1) Wasserstoffatom,
2) -(C₁-C₆)-Alkyl,
3) Phenyl oder
4) -(C₃-C₆)-Cycloalkyl, steht,
R2 und R3 gleich oder verschieden sind und unabhängig voneinander für Wasserstoffatom oder
-(C₁-C₄)-Alkyl, stehen,
das dadurch gekennzeichnet ist, dass man ein Boronoindol der Formel IV, worin R1 wie für Formel I definiert ist,
R4 und R5 gleich oder verschieden sind und unabhängig voneinander für -OH oder
-(C₁-C₆)-Alkyl, stehen, oder
R4 und R5 bilden zusammen mit dem B-Atom an das sie gebunden sind einen Ring aus der Reihe Borolan, Borinan, Borepan, Borocan, [1,3,2]Dioxaborolan, [1,3,2]Dioxaborinan, [1,3,2]Dioxaborepan, [1,3,2]Dioxaborocan oder [1,3,6,2]Dioxazaborocan,
P für
1) Wasserstoffatom,
2) -C(O)-O-R6, worin R6 für
   a) Wasserstoffatom,
   b) -(C₁-C₆)-Alkyl,
   c) -(C₆-C₁₄)-Aryl, wobei Aryl ausgewählt ist aus der Reihe Phenyl, Naphthyl, Anthryl oder Fluorenyl und worin Aryl unsubstituiert oder ein, zwei oder dreifach durch -(C₁-C₆)-Alkyl substituiert ist,
   d) -(C₃-C₆)-Cycloalkyl oder
   e) für einen Rest aus der Reihe Acridinyl, Azepinyl, Azetidinyl, Aziridinyl, Benzimidazalinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4αH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxodiazolyl, 1,3,4-Oxadiazolyl, Oxazolidinyl, Oxazolyl, Oxazolidinyl, Oxothiolanyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Thiomorpholinyl, Thiophenyl, Triazinyl, 1,2,3-Triazolyl, 1,2,3-Triazolyl, 1,2,4-Triazolyl, 1,2,5-Triazolyl, 1,3,4-Triazolyl oder Xanthenyl, steht,
3) -SO₃-R6,
4) -O-SO₂-R6,
5) -Si-R6 oder
6) Benzyl steht,
mit einem Pyrimidin der Formel VI, worin
X für Cl, Br, I, -O-Tosylat, -O-Mesylat oder-O-Acetat steht, und
Y für Cl, Br, I, -O-Tosylat, -O-Mesylat oder -O-Acetat steht,
zu einer Verbindung der Formel VII umsetzt und anschließend die Verbindung der Formel VII mit einem Amin der Formel VIII, worin R2 und R3 gleich oder verschieden sind und unabhängig voneinander für Wasserstoffatom oder -(C₁-C₄)-Alkyl stehen, zu einer Verbindung der Formel I umsetzt und die gegebenenfalls vorhandene N-Schutzgruppe abspaltet.

Die Erfindung betrifft ferner ein Verfahren zur Gewinnung der Verbindung der Formel I, wobei
R1 für Wasserstoffatom oder Ethyl steht,
R2 und R3 gleich oder verschieden sind und unabhängig voneinander für Wasserstoffatom oder
-(C₁-C₄)-Alkyl, stehen,
das dadurch gekennzeichnet ist, dass man ein Boronoindol der Formel IV, worin R1 wie für Formel I definiert ist,
R4 und R5 gleich sind und für-OH stehen,
P für
1) Wasserstoffatom,
2) -O-Tosylat oder
3) Benzyl steht,
mit einem Pyrimidin der Formel VI, worin
X für Cl, Br, I, -O-Tosylat, -O-Mesylat oder-O-Acetat steht, und
Y für Cl, Br, I, -O-Tosylat, -O-Mesylat oder -O-Acetat steht,
zu einer Verbindung der Formel VII umsetzt und anschließend die Verbindung der Formel VII mit einem Amin der Formel VIII, worin R2 und R3 gleich oder verschieden sind und unabhängig voneinander für Wasserstoffatom oder-(C₁-C₄)-Alkyl stehen, zu einer Verbindung der Formel I umsetzt und die gegebenenfalls vorhandene N-Schutzgruppe abspaltet.

Der Verfahrensschritt a) wird beispielsweise gemäß den Reaktionsbedingungen wie sie von B. Jiang und C. Yang in Heterocycles, Vol. 53, 2000, S. 1489-1498 beschrieben sind, durchgeführt.
Die Umsetzung der Boronoindole (IV) mit den Pyrimidinderivaten (V) wird bevorzugt in Gegenwart katalytischer Mengen an Palladium- oder Nickelverbindungen wie Pd(PPh₃)₄, Pd₂(dba)₃, Pd(OAc)₂ oder PdCl₂/TPPTS durchgeführt.
   Die Reaktionstemperatur beträgt dabei von 40 °C bis 80 °C, bevorzugt von 60 °C bis 70 °C. Die Reaktionszeit liegt im allgemeinen von 2 bis 3 Stunden, je nach Zusammensetzung des Gemisches und des gewählten Temperaturbereiches. Als Lösungsmittel eignen sich beispielsweise Methanol, Ethanol oder Toluol. Das molare Verhältnis der Verbindung der Formel IV zur Verbindung der Formel V liegt beispielsweise bei 1:1 bis 1:1,3.
   Die Reinheit wird durch HPLC ermittelt.
Die Verbindungen der Formel IV sind entweder bekannt oder lassen sich beispielsweise durch Umsetzung der entsprechenden N-geschützten Indol-5-carbonsäurederivate (XI) mit Basen wie LDA, LiTMP oder LiHMDS und anschließende Umsetzung mit Borsäureestern wie Triisopropylborat oder Trimethylborat herstellen (Schema 5). 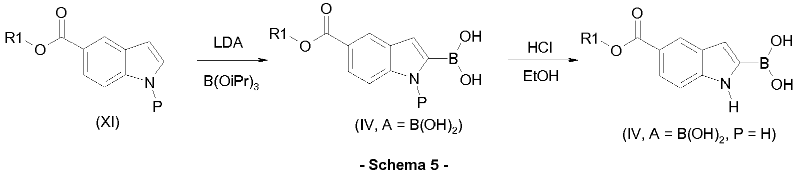
Es hat sich als vorteilhafter erwiesen, vor der eigentlichen Umsetzung die N-Schutzgruppe zu entfernen, da in diesem Fall das während der Kupplung durch Abspaltung der Borono-Gruppe als Nebenprodukt entstehende Indol (XI) in geringerem Maße gebildet wird, was in einer höheren Ausbeute resultiert.
Die Verbindungen der Formel V sind entweder bekannt oder lassen sich durch Umsetzung von Pyrimidinderivaten (VI) mit Aminen VIII und anschließende Abtrennung vom Isomeren XII, beispielsweise durch Chromatographie oder Wasserdampfdestillation, herstellen (siehe Schema 6)

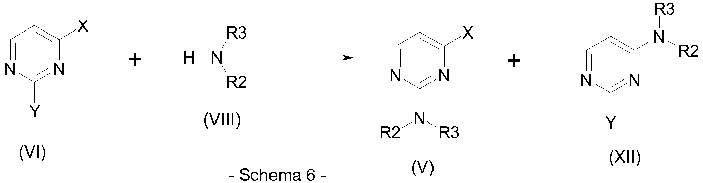

Es ist auch möglich, die eigentliche Umsetzung mit Gemischen von (V) und (XII) durchzuführen, da sich überraschenderweise gezeigt hat, dass das Isomere (XII) unter den Reaktionsbedingungen dieser Kupplung nicht zu dem zu (I) isomeren (XIII) reagiert (Schema 7)

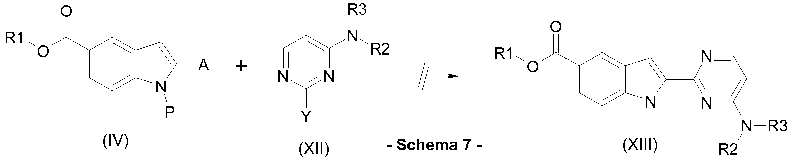

Der Verfahrensschritt b) wird analog zu a) durchgeführt

Der Verfahrensschritt c) wird beispielsweise gemäß den Reaktionsbedingungen wie sie von I. Beletskaya et al in Tetrahedron Letters 44 (2003) 501-5013 beschrieben sind, durchgeführt.
Die Umsetzung der Verbindungen der Formel IX mit den Pyrimidinderivaten (V) erfolgt bevorzugt in Gegenwart von Metallkatalysatoren wie Pd(OAc)₂ oder Cul und Triphenylphosphin oder TPPTS. Der Ringschluß von (X) zu (I) kann beispielsweise durch Zugabe von Basen wie KOtBu oder KHMDS erfolgen.
Die Reaktionstemperatur beträgt dabei von 15 °C bis 30 °C, bevorzugt von 20 °C bis 25 °C. Die Reaktionszeit liegt im allgemeinen von 20 bis 24 Stunden, je nach Zusammensetzung des Gemisches und des gewählten Temperaturbereiches. Als Lösungsmittel eignen sich beispielsweise N-Methylpyrrolidon (NMP) oder Dimethyformamid (DMF).
Die Reinheit wird durch Hochdruckflüssigchromatographie (HPLC) ermittelt.

Die Herstellung physiologisch verträglicher Salze aus zur Salzbildung befähigten Verbindungen der Formel I, einschließlich deren stereoisomeren Formen, gemäß Verfahrensschritt d) erfolgt in an sich bekannter Weise. Die Verbindungen der Formel I bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin, Diethanolamin oder Triethanolamin, Trometamol oder auch basischen Aminosäuren, etwa Lysin, Ornithin oder Arginin, stabile Alkali-, Erdalkali- oder gegebenenfalls substituierte Ammoniumsalze. Sofern die Verbindungen der Formel I basische Gruppen aufweisen, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Hemischwefel-, Phosphor-, Methansulfon-, Benzolsulfon-, p-Toluolsulfon-, 4-Brombenzol-sulfon-, Cyclohexylamidosulfon-, Trifluormethylsulfon-, 2-Hydroxyethansulfon-, Essig-, Oxal-, Wein-, Bernstein-, Glycerolphosphor-, Milch-, Äpfel-, Adipin-, Citronen-, Fumar-, Malein-, Glucon-, Glucuron-, Palmitin-, oder Trifluoressigsäure in Frage. Bevorzugt sind Alkali- und Erdalkalimetalle oder Ammoniumsalze. Beispiele für Alkali- und Erdalkalimetalle sind Li, Na, K, Rb, Cs, Be, Mg, Ca, Sr oder Ba. Beispiel für Ammoniumsalz ist NH₄.

Die Erfindung betrifft ferner ein Verfahren zur Gewinnung der Verbindung der Formel I, das dadurch gekennzeichnet ist, dass man
a) eine Aminobenzoesäure der Formel XIV 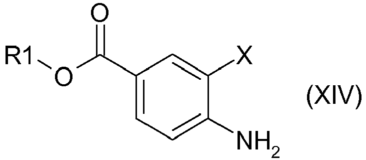 wobei R1 wie in Formel I und X wie in Formel V definiert sind
   mit Trimethylsilylacetylen zu einer Verbindung der Formel XVI umsetzt, 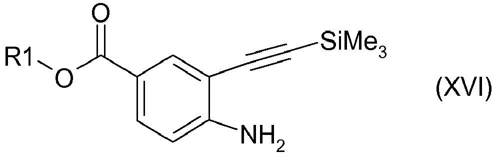 wobei R1 wie in Formel I definiert ist und Me für Methyl steht, und
b) die Verbindung der Formel XVI zu einem Alkin der Formel IX umsetz, wobei
   R1 wie in Formel I definiert ist, und
c) das Alkin der Formel IX mit einem Pyrimidin der Formel (V) zu einem Alkin der Formel X umsetzt,

   worin R1, R2 und R3 die gleiche Bedeutung wie in Formel I haben, und die Verbindung der Formel X durch Ringschluss in die Verbindung der Formel I überführt.

Die Verbindungen der Formel IX sind entweder bekannt oder lassen sich durch Umsetzung von Aminobenzoesäurederivaten (XIV) mit Trimethylsilylacetylen (XV) und anschließender Abspaltung der Silylschutzgruppe hergestellen (Schema 8) 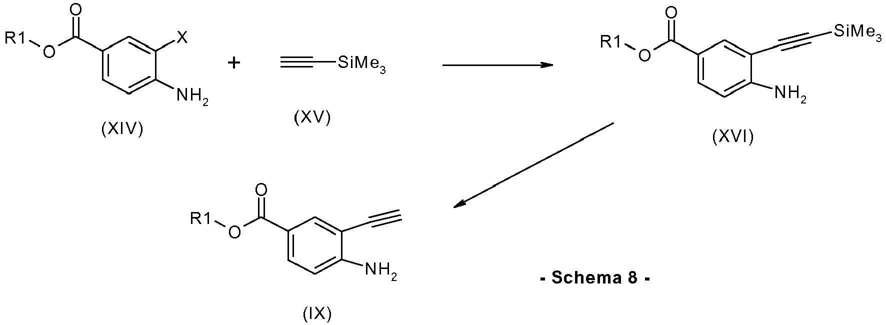 Unter dem Begriff "N-Schutzgruppe" für P werden übliche Aminschutzgruppen, wie sie in T. Greene, "Protective Groups in Organic Synthesis" beschrieben sind, verstanden. Weitere Beispiele für N-Schutzgruppen sind die Reste 2) bis 6) für P als Rest in der Verbindung der Formel II.
Unter dem Begriff "katalytischer Mengen an Palladium- oder Nickelverbindungen" werden von 0,03 bis 0,3 Mol (z.B. bezogen auf Mol der Verbindung der Formel IV) der folgenden Verbindungen Pd(PPh₃)₄, Pd₂(dba)₃, Pd(OAc)₂ oder PdCl₂/TPPTS verstanden.

Unter dem Begriff "Metallkatalysatoren" werden zum Beispiel von 0,03 bis 0,3 Mol (beispielsweise bezogen auf Mol der Verbindung der Formel IV) der folgender Verbindungen Pd(PPh₃)₄, Pd₂(dba)₃, Pd(OAc)₂ oder PdCl₂/TPPTS verstanden.
Unter den Begriffen "-(C₁-C₁₂)-Alkyl" werden Kohlenwasserstoffreste verstanden, deren Kohlenstoffkette geradkettig oder verzweigt ist und 1 bis 12 Kohlenstoffatome enthält. Beispiele sind Methyl, Ethyl, Propyl, Iso-Propyl, Butyl, Iso-Butyl, tertiär-Butyl, Pentyl, Neopentyl, Hexyl, 2,3-Dimethylbutan, Neohexyl, Heptyl, Octyl, Nonanyl, Decanyl oder Dodecanyl.
Unter dem Begriff "(C₃-C₈)-Cycloalkyl" werden Reste verstanden wie Verbindungen, die sich von 3- bis 8-gliedrige Monocyclen wie Cyclopropyl, Cyclobutyl, Cyclopentyl, Cyclohexyl, Cycloheptyl oder Cyclooctyl herleiten. Unter den Begriffen "-(C₆-C₁₄)-Aryl," oder "Aryl" werden aromatische Kohlenstoffreste verstanden mit 6 bis 14 Kohlenstoffatomen im Ring. -(C₆-C₁₄)-Arylreste sind beispielsweise Phenyl, Naphthyl, zum Beispiel 1-Naphthyl, 2-Naphthyl, Anthryl oder Fluorenyl. Naphthylreste und insbesondere Phenylreste sind bevorzugte Arylreste.
Unter dem Begriff "4- bis 15-gliedriger Het-Ring" werden Ringsysteme verstanden mit 4 bis 15 Kohlenstoffatomen, die in ein, zwei oder drei miteinander verbundenen Ringsystemen vorliegen und die ein, zwei, drei oder vier gleiche oder verschiedene Heteroatome aus der Reihe Sauerstoff, Stickstoff oder Schwefel enthalten. Beispiele für diese Ringsysteme sind die Reste Acridinyl, Azepinyl, Azetidinyl, Aziridinyl, Benzimidazalinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4αH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydro-chinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzo-furanyl, Isochromanyl, Isoindazolyl, Isoindolinyl, Isoindolyl, Isochinolinyl, Benzimidazolyl, Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxodiazolyl, 1,2,4-Oxodiazolyl, 1,2,5-Oxadiazolyl, 1,3,4-Oxadiazolyl, Oxazolidinyl, Oxazolyl, Oxazolidinyl, Oxothiolanyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Thiomorpholinyl, Thiophenyl,
   Triazinyl, 1,2,3-Triazolyl, 1,2,3-Triazolyl, 1,2,4-Triazolyl, 1,2,5-Triazolyl, 1,3,4-Triazolyl und Xanthenyl.
Unter dem Begriff "R4 und R5 bilden zusammen mit dem B-Atom an das sie gebunden sind einen Ring mit 4, 5, 6 oder 7 Kohlenstoffatomen im Ring und der Ring kann statt der jeweiligen Kohlenstoffatomen zwei Sauerstoffatome oder zwei Sauerstoffatome und ein Stickstoffatom enthalten," werden Ringsysteme verstanden, die sich beispielsweise von Borolan, Borinan, Borepan, Borocan, [1,3,2]Dioxaborolan, [1,3,2]Dioxaborinan, [1,3,2]Dioxaborepan, [1,3,2]Dioxaborocan oder [1,3,6,2]Dioxazaborocan ableiten.

Die Erfindung betrifft ferner neue Verbindungen der Formel II, 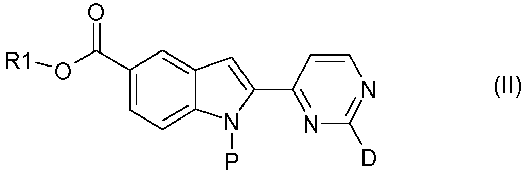 oder ein physiologisch verträgliches Salz der Verbindung der Formel II, wobei
R1 für
   1) Wasserstoffatom,
   2) -(C₁-C₁₂)-Alkyl,
   3) -(C₆-C₁₄)-Aryl,
   4) -(C₃-C₈)-Cycloalkyl oder
   5) 4- bis 15-gliedriger Het-Ring, steht,
   mit der Maßgabe, dass R1 nicht Wasserstoffatom ist, wenn P für Wasserstoffatom steht und D für -N(R2)-R3 steht, worin R2 für Wasserstoffatom steht und R3 für -(C₁-C₄)-Alkyl steht,
P für
   1) Wasserstoffatom,
   2) -C(O)-O-R6, worin R6 für
      a) Wasserstoffatom,
      b) -(C₁-C₁₂)-Alkyl,
      c) -(C₆-C₁₄)-Aryl, wobei Aryl unsubstituiert oder ein, zwei oder dreifach durch -(C₁-C₆)-Alkyl substituiert ist,
      d) -(C₃-C₈)-Cycloalkyl oder
      e) 4- bis 15-gliedriger Het-Ring steht,
   3) -SO₃-R6,
      4) -O-SO₂-R6,
      5) -Si-R6 oder
      6) Benzyl steht,
D für
   1) -N(R2)-R3,
   2) Halogen,
   3) -O-SO₂-R2 oder
   4) -O-C(O)-R2 steht,
   worin R2 und R3 gleich oder verschieden sind und unabhängig voneinander für
   a) Wasserstoffatom,
   b) -(C₁-C₁₂)-Alkyl,
   c) -(C₆-C₁₄)-Aryl, wobei Aryl unsubstituiert oder ein, zwei oder dreifach durch -(C₁-C₆)-Alkyl substituiert ist,
   d) -(C₃-C₈)-Cycloalkyl oder
   e) 4- bis 15-gliedriger Het-Ring, stehen.

Ein weiterer Aspekt der Erfindung betrifft neue Verbindungen der Formel II, worin
R1 für
1) Wasserstoffatom,
2) -(C₁-C₆)-Alkyl,
3) Phenyl oder
4) -(C₃-C₆)-Cycloalkyl, steht,
mit der Maßgabe, dass R1 nicht Wasserstoffatom ist, wenn P für Wasserstoffatom steht und D für -N(R2)-R3 steht, worin R2 für Wasserstoffatom steht und R3 für-(C₁-C₄)-Alkyl steht,
D für Chlor, Brom, Jod, Fluor oder -N(R2)-R3 steht, wobei
R2 und R3 gleich oder verschieden sind und unabhängig voneinander für Wasserstoffatom oder-(C₁-C₄)-Alkyl, stehen,
P für
1) Wasserstoffatom,
2) -C(O)-O-R6, worin R6 für
   a) Wasserstoffatom,
   b) -(C₁-C₆)-Alkyl,
   c) -(C₆-C₁₄)-Aryl, wobei Aryl ausgewählt ist aus der Reihe Phenyl, Naphthyl, Anthryl oder Fluorenyl und worin Aryl unsubstituiert oder ein, zwei oder dreifach durch -(C₁-C₆)-Alkyl substituiert ist,
   d) -(C₃-C₆)-Cycloalkyl oder
   e) für einen Rest aus der Reihe Acridinyl, Azepinyl, Azetidinyl, Aziridinyl, Benzimidazalinyl, Benzimidazolyl, Benzofuranyl, Benzothiofuranyl, Benzo-thiophenyl, Benzoxazolyl, Benzthiazolyl, Benztriazolyl, Benztetrazolyl, Benz-isoxazolyl, Benzisothiazolyl, Carbazolyl, 4αH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl, Benzimidazolyl, Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxodiazolyl, Oxazolidinyl, Oxazolyl, Oxazolidinyl, Oxothiolanyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimida-zolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroiso-chinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Thiomorpholinyl, Thiophenyl, Triazinyl, 1,2,3-Triazolyl, 1,2,3-Triazolyl, 1,2,4-Triazolyl, 1,2,5-Triazolyl, 1,3,4-Triazolyl oder Xanthenyl, steht,
3) -SO₃-R6,
4) -O-SO₂-R6,
5) -Si-R6 oder
6) Benzyl steht.

Ein weiterer Aspekt der Erfindung betrifft neue Verbindungen der Formel II, worin
R1 für Wasserstoffatom oder Ethyl steht,
mit der Maßgabe, dass R1 nicht Wasserstoffatom ist, wenn P für Wasserstoffatom steht und D für -N(R2)-R3 steht, worin R2 für Wasserstoffatom steht und R3 für -(C₁-C₄)-Alkyl steht,
D für Chlor oder -N(R2)-R3 steht, wobei
R2 und R3 gleich oder verschieden sind und unabhängig voneinander für Wasserstoffatom oder -(C₁-C₄)-Alkyl, stehen,
P für Wasserstoffatom, -O-Tosylat oder Benzyl steht.

Die Verbindungen der Formel II sind erhältlich, beispielsweise durch die Verfahrensvarianten a), c) oder d) zur Herstellung der Verbindung der Formel I oder ergeben sich als Zwischenverbindungen der Formel VII in der Verfahrensvariante b) zur Herstellung der Verbindung der Formel I.

Die Erfindung betrifft ferner neue Verbindungen der Formel IV, 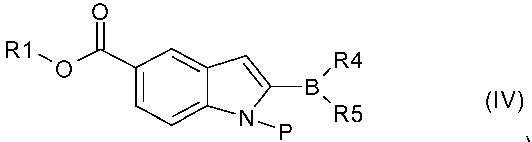 worin
R1 für
   1) Wasserstoffatom,
   2) -(C₁-C₁₂)-Alkyl,
   3) -(C₆-C₁₄)-Aryl,
   4) -(C₃-C₈)-Cycloalkyl oder
   5) 4- bis 15-gliedriger Het-Ring, steht,
R4 und R5 gleich oder verschieden sind und unabhängig voneinander für
   1) -OH,
   2) -O-(C₁-C₁₂)-Alkyl,
   3) -O-(C₆-C₁₄)-Aryl,
   4) -O-(C₃-C₈)-Cycloalkyl,
   5) -(C₁-C₁₂)-Alkyl oder
   6) -O-Het, wobei Het ein 4- bis 15-gliedriger Het-Ring bedeutet, stehen, oder
R4 und R5 bilden zusammen mit dem B-Atom an das sie gebunden sind einen Ring mit 4, 5, 6 oder 7 Kohlenstoffatomen im Ring und der Ring kann statt der jeweiligen Kohlenstoffatome zwei Sauerstoffatome oder zwei Sauerstoffatome und ein Stickstoffatom enthalten,
P für
   1) Wasserstoffatom,
   2) -C(O)-O-R6, worin R6 für
      a) Wasserstoffatom,
      b) -(C₁-C₁₂)-Alkyl,
      c) -(C₆-C₁₄)-Aryl, wobei Aryl unsubstituiert oder ein, zwei oder dreifach durch -(C₁-C₆)-Alkyl substituiert ist,
      d) -(C₃-C₈)-Cycloalkyl oder
      e) 4- bis 15-gliedriger Het-Ring steht,
   3) -SO₃-R6,
   4) -O-SO₂-R6,
   5) -Si-R6 oder
   6) Benzyl steht,
   mit der Maßgabe, dass R4 und R5 nicht -OH oder-O-(C₁-C₁₂)-Alkyl sind, wenn P für
   -C(O)-O-(C₁-C₁₂)-Alkyl steht oder R1 für- (C₁-C₁₂)-Alkyl steht.

Die Erfindung betrifft ferner neue Verbindungen der Formel IV, wobei R1 für
1) Wasserstoffatom,
2) -(C₁-C₈)-Alkyl,
3) -(C₆-C₁₄)-Aryl, wobei Aryl ausgewählt ist aus der Reihe Phenyl, Naphthyl, Anthryl oder Fluorenyl,
4) -(C₃-C₆)-Cycloalkyl oder
5) 4- bis 15-gliedriger Het-Ring worin Het für einen Rest aus der Reihe Acridinyl, Azepinyl, Azetidinyl, Aziridinyl, Benzimidazalinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4αH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1 H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxodiazolyl, 1,2,4-Oxodiazolyl, 1,2,5-Oxodiazolyl, 1,3,4-Oxodiazolyl, Oxazolidinyl, Oxazolyl, Oxazolidinyl, Oxothiolanyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Thiomorpholinyl, Thiophenyl, Triazinyl, 1,2,3-Triazolyl, 1,2,3-Triazolyl, 1,2,4-Triazolyl, 1,2,5-Triazolyl, 1,3,4-Triazolyl oder Xanthenyl steht,
R4 und R5 gleich oder verschieden sind und unabhängig voneinander für
1) -OH,
2) -O-(C₁-C₁₂)-Alkyl,
3) -O-(C₆-C₁₄)-Aryl, worin Aryl wie oben definiert ist,
4) -O-(C₃-C₆)-Cycloalkyl,
5) -(C₁-C₆)-Alkyl oder
6) -O-Het, wobei Het ein 4- bis 15-gliedriger Het-Ring bedeutet und wie oben definiert ist, stehen, oder
R4 und R5 bilden zusammen mit dem B-Atom an das sie gebunden sind einen Ring aus der Reihe Borolan, Borinan, Borepan, Borocan, [1,3,2]Dioxaborolan, [1,3,2]Dioxaborinan, [1,3,2]Dioxaborepan, [1,3,2]Dioxaborocan oder [1,3,6,2]Dioxazaborocan, und
P für Wasserstoffatom steht.

Die Erfindung betrifft ferner neue Verbindungen der Formel X, 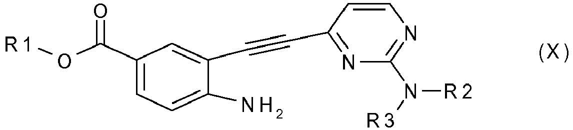 wobei
R1 für
   1) Wasserstoffatom,
   2) -(C₁-C₁₂)-Alkyl,
   3) -(C₆-C₁₄)-Aryl,
   4) -(C₃-C₈)-Cycloalkyl oder
   5) 4- bis 15-gliedriger Het-Ring, steht,
R2 und R3 gleich oder verschieden sind und unabhängig voneinander für
   1) Wasserstoffatom,
   2) -(C₁-C₁₂)-Alkyl,
   3) -(C₆-C₁₄)-Aryl, wobei Aryl unsubstituiert oder ein, zwei oder dreifach durch
      -(C₁-C₆)-Alkyl substituiert ist,
   4) -(C₃-C₈)-Cycloalkyl oder
   5) 4- bis 15-gliedriger Het-Ring, stehen.

Die Verbindungen der Formel X sind erhältlich, beispielsweise durch die Verfahrensvariante c) zur Herstellung der Verbindung der Formel I.

Die Erfindung betrifft ferner neue Verbindungen der Formel XVI, 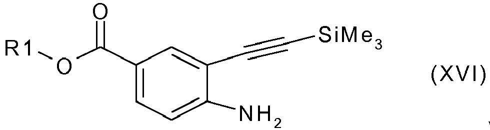 wobei R1 für
1) Wasserstoffatom,
2) -(C₁-C₁₂)-Alkyl,
3) -(C₆-C₁₄)-Aryl,
4) -(C₃-C₈)-Cycloalkyl oder
5) 4- bis 15-gliedriger Het-Ring, steht.

Die Verbindungen der Formeln II, IV, X, XI oder XVI eignen sich als Zwischenverbindungen zur Herstellung von IkB-Kinase Inhibitoren wie sie beispielsweise in WO 01/30774 A1 beschrieben werden.

Nachfolgend ist die Erfindung an Hand von Beispielen näher erläutert. Endprodukte werden in der Regel durch ¹H-NMR (400 MHz, in DMSO-D6) bestimmt. Temperaturangaben in Grad Celsius, RT bedeutet Raumtemperatur (22 °C bis 26 °C). Verwendete Abkürzungen sind entweder erläutert oder entsprechen den üblichen Konventionen.

### Liste der Abkürzungen:

- Dba: Dibenzylidenaceton
- KHMDS: Kaliumhexamethyldisilazid
- KOtBu: Kaliumtertiär-butylat
- LDA: Lithiumdiisopropylamid
- LiHMDS: Lithiumhexamethyldisilazid
- LiTMP: Lithiumtetramethylpiperazid
- OTos: 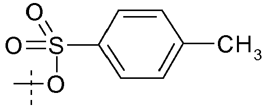 BBBentspricht -O-Tosylat
- OMes: 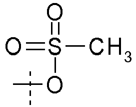 entspricht -O-Mesylat
- OAc: 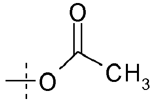 entspricht -O-Acetat
- Pd(PPh₃)₄: Tetrakis(triphenylphosphin)palladium(O)
- Tos: 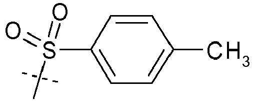 entspricht Tosylat
- TPPTS: Triphenylphosphintrisulfonat
- THF: Tetrahydrofuran

### Beispiel 1: Synthese von 2-(2-Chloropyrimidin-4-yl)-1H-indol-5-carbonsäureethylester

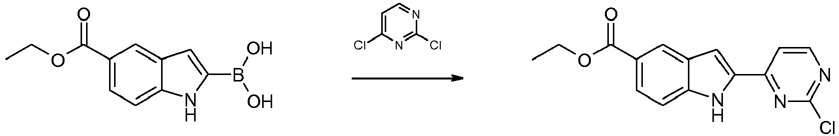

28 g (114 mmol) 2-Borono-5-ethoxycarbonylindol, 12 g (113 mmol) Natriumcarbonat und 17,2 g 2,4-(113 mmol) Dichloropyrimidin wurden in 412 ml Ethanol vorgelegt. Die klare Lösung wurde durch kräftiges Rühren und Durchleiten von Argon (20 Minuten) von Sauerstoff befreit. Bei RT wurden 2,67 g Terakis(triphenylphosphin)palladium(0) zugegeben. Das Gemisch wurde 2 Stunden (h) auf 65 °C bis 70 °C erhitzt. Anschließend wurden 112 ml Wasser und 112 ml 30 %ige Salzsäure zugeben und auf 0 °C abgekühlt. Nach Filtration und trocknen unter verminderten Druck wurden 37,3 g (93% der Theorie) 2-(2-Chloropyrimidin-4-yl)-1H-indol-5-carbonsäureethylester erhalten (HPLC > 96%). Die Bestimmung der Reinheit erfolgte durch Hochdruckflüssigchromatographie (HPLC):

| | | | | | |
|---|---|---|---|---|---|
| Säule: | Waters Symetry Shield RP8 3,9 * 150 | | | | |
| Temperatur: | 40 °C | | | | |
| Fluss: | 1 mL/ min | | | Injektionsvolumen: | 10 µL |
| Druck: | | 90 bar | | UV: | 254 nm |
| Eluent: | | A: Wasser/ Trifluoressigsäure (0,05%) | | | |
| | B: Acetonitril/ Trifluoressigsäure (0,05%) | | | | |
| Zeit (min) | 0 | 15 | 20 | 25 | 30 |
| A(%) | 80 | 25 | 25 | 80 | 80 |
| B(%) | 20 | 75 | 75 | 20 | 20 |
| Retentionszeit Titelverbindung: 12,6 min | | | | | |

### Beispiel 2: Synthese von 2-(2-Methylaminopyrimidin-4-yl)-1H-indol-5-carbonsäureethylester

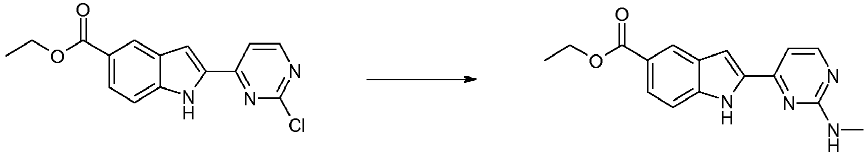

Es wurden 30 g (95,4 mmol) 2-(2-Chloropyrimidin-4-yl)-1H-indol-5-carbonsäureethylester vorgelegt und in 150 ml Ethanol suspendiert. Zu dieser Suspension wurden 53,9 g Methylamin-Lösung in Ethanol (8 M) gegeben und im Autoklaven 4 h auf 75 °C bis 80 °C erhitzt. Nach Einengen und Waschen mit Ethanol wurden 29,7 g 2-(2-Methylaminopyrimidin-4-yl)-1 H-indol-5-carbonsäureethylester erhalten (97.6 HPLC-FI.-%). LCMS: [M + H]⊕ 297,12
HPLC Methode wie in Beispiel 1; Retentionszeit Titelverbindung: 5,8 min

### Beispiel 3: Synthese von 2-(2-Methylaminopyrimidin-4-yl)-1H-indol-5-carbonsäure -Natriumsalz

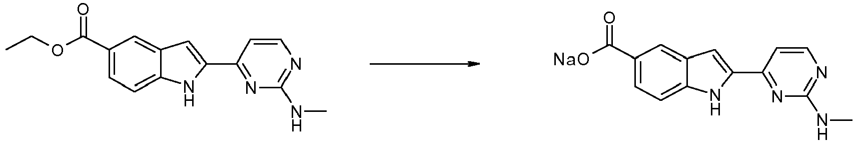

25 g 2-(2-Methylominopyrimidin-4-yl)-1H-indol-5-carbonsäureethylester wurden mit 200 ml Ethanol und 24,5 g 33 %iger Natronlauge versetzt und 4 h auf 65°C bis 70 °C erhitzt. Nach dem Abkühlen wurde abgesaugt und der Niederschlag wurde mit 15 ml Ethanol/Wasser (9:1) gewaschen. Es wurden 24,5 g (87,6 % der Theorie) 2-(2-Methylaminopyrimidin-4-yl)-1H-indol-5-carbonsäure -Natriumsalz erhalten (98.1 HPLC-Flächen-%). LCMS: [M + H]⊕ 269,10
HPLC Methode wie in Beispiel 1; Retentionszeit Titelverbindung: 3,3 min

### Beispiel 4: Synthese von 4-Amino-3-trimethylsilylethinyl-benzoesäuremethylester

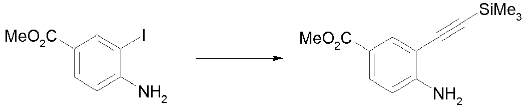

5,83 g (20 mmol) 4-Aminobenzoesäuremethylester, 20,2 g (198 mmol) Triethylamin und 80 ml Toluol wurden vorgelegt. Die klare Lösung wurde durch kräftiges Rühren und
Durchleiten von Argon (20 Minuten) von Sauerstoff befreit. Bei einer Innentemperatur von
20 °C wurden 3,2 g (33 mmol) Trimethylsilylacetylen, 76 mg Kupfer(I)iodid und 52 mg Triphenylphosphin zugegeben. Nach wässriger Aufarbeitung erhielt man 5,45 g 4-Amino-3-trimethylsilylethinyl-benzoesäuremethylester (HPLC: > 99 Fläche-%). HPLC Methode wie in Beispiel 1.

### Beispiel 5: Synthese von 4-Amino-3-ethinylbenzoesäuremethylester

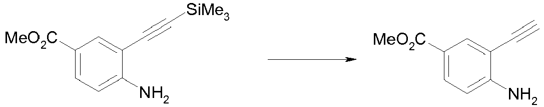

1,9 g (7,7 mmol) 4-Amino-3-trimethylsilylethinyl-benzoesäuremethylester wurden in 20 ml Tetrahydrofuran (THF) vorgelegt. Bei 5°C bis 8 °C wurde innerhalb von 5 Minuten 8,45 ml (8,5 mmol) Tetrabutylammoniumfluorid-Lösung (1 M in THF) zugetropft. Nach 25 min bei 2 °C wurden 438 ml Essigsäure zugegeben. Nach Zugabe von Wasser und Extraktion mit Dichlormethan erhielt man nach Entfernung des Lösungsmittels 1,35 g 4-Amino-3-ethinylbenzoesäuremethylester.
HPLC Methode wie in Beispiel 1

### Beispiel 6: Synthese von 4-Amino-3-(1-methylamino-pyrimidin-4-yl)-ethinylbenzoesäuremethyl-ester

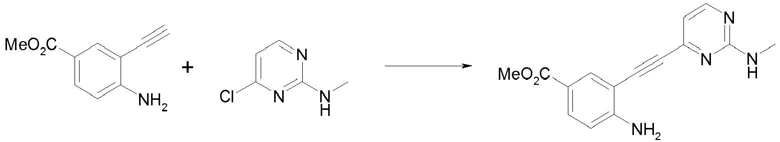

3,0 g (17 mmol) 4-Amino-3-ethinylbenzoesäuremethylester und 2,6 g (19 mmol) 4-Chlor-2-methylaminopyrimidin wurden in 20 ml Dimethyformamid (DMF) und 8,7 g (85 mmol) Triethylamin vorgelegt und 5 min mit Argon unter Rühren entgast. Anschließend wurden 65 mg Kupfer(I)iodid und 200 mg Tetrakis(triphenylomin)polladium(0) zugegeben und die Mischung 3 h auf 71 °C erhitzt. Nach wässriger Aufarbeitung erhielt man 4,1 g 4-Amino-3-(1-methylamino-pyrimidin-4-yl)-ethinylbenzoesäuremethylester. (HPLC: 99,7 Flächen-%)
HPLC Methode wie in Beispiel 1.

### Beispiel 7: Synthese von 2-(2-Methylaminopyrimidin-4-yl)-1H-indol-5-carbonsäuremethyl-ester durch Cyclisierung von 4-Amino-3-(1-methylamino-pyrimidin-4-yl)-ethinylbenzoesäure-methylester

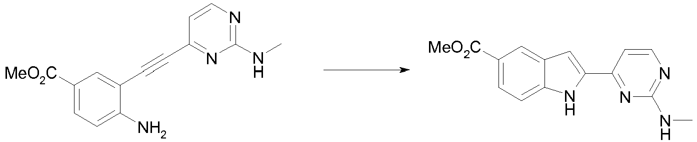

73 mg (0,7 mmol) Kalium-tert-butylat wurden in 1 ml NMP gelöst und mit einer Lösung von 140 mg (0,5 mmol) 4-Amino-3-(1-methylamino-pyrimidin-4-yl)-ethinylbenzoesäuremethylester in 1 ml NMP versetzt. Anschließend wurde 24 h bei RT nachgerührt. Nach wässriger Aufarbeitung erhielt man 115 mg 2-(2-Methylaminopyrimidin-4-yl)-1H-indol-5-carbonsäuremethylester (HPLC: 92,3 Fläche-%).

### Beispiel 8: Synthese von 2-Borono-5-ethoxycarbonylindol

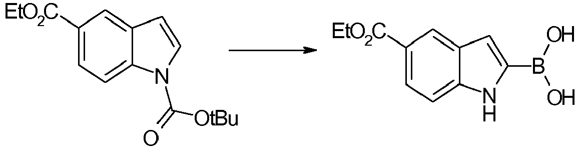

150 g (519 mmol) N-Boc-5-ethoxycarbonylindol und 192 ml (833 mmol) Triisopropylborat in 350 ml Toluol wurden bei 5 °C bis 10 °C mit 350 ml einer 1,8 molaren Lösung von LDA in THF versetzt. Es wurde 5 min nachgerührt und die Reaktionsmischung zu einer Lösung von 278 g 30 %iger Salzsäure und 940 ml Wasser gegeben. Anschließend wurde 30 min bei 5 °C bis 10 °C gerührt. Danach wurde filtriert und der Filterkuchen in 530 ml Ethanol suspendiert. Diese Suspension wurde bei 40 °C zu einer Lösung von 500 ml 30 %iger Salzsäure und 224 ml Ethanol gegeben. Anschließend wurde 2,5 h bei 40 °C bis 45 °C gerührt und bei 30 °C mit 380 ml Wasser versetzt. Dann wurde auf 10 °C bis 15 °C abgekühlt, 30 min bei dieser Temperatur nachgerührt und filtriert. Nach Trocknung unter vermindertem Druck wurden 79,5 g (61 % der Theorie) 2-Borono-5-ethoxycarbonylindol erhalten (HPLC: 92.7 Fl.-%).

## Patentansprüche

1. Verfahren zur Gewinnung der Verbindung der Formel I, 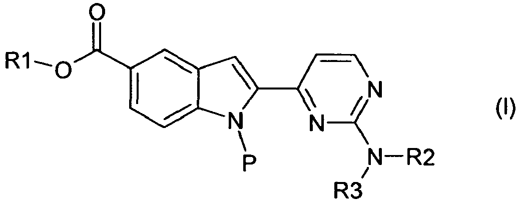 worin
R1 für
1) ein Wasserstoffatom,
2) -(C₁-C₁₂)-Alkyl,
3) -(C₆-C₁₄)-Aryl,
4) -(C₃-C₈)-Cycloalkyl oder
5) 4- bis 15-gliedriger Het-Ring, steht,
R2 und R3 gleich oder verschieden sind und unabhängig voneinander für
1) ein Wasserstoffatom,
2) -(C₁-C₁₂)-Alkyl,
3) -(C₆-C₁₄)-Aryl, wobei Aryl unsubstituiert oder ein-, zwei- oder dreifach durch -(C₁-C₆)-Alkyl substituiert ist,
4) -(C₃-C₈)-Cycloalkyl oder
5) 4- bis 15-gliedriger Het-Ring, stehen,
P für ein Wasserstoffatom steht,
**dadurch gekennzeichnet ist, dass** man
a) ein Boronoindol der Formel IV 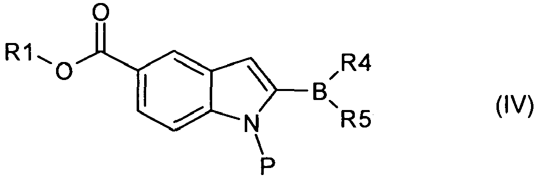 worin
R1 die gleiche Bedeutung wie in Formel I hat,
R4 und R5 gleich oder verschieden sind und unabhängig voneinander für
1) -OH,
2) -O-(C₁-C₁₂)-Alkyl,
3) -O-(C₆-C₁₄)-Aryl,
4) -O-(C₃-C₈)-Cycloalkyl,
5) -(C₁-C₁₂)-Alkyl oder
6) -O-Het, wobei Het ein 4- bis 15-gliedriger Het-Ring bedeutet, stehen, oder
R4 und R5 bilden zusammen mit dem B-Atom, an das sie gebunden sind, einen Ring mit 4, 5, 6 oder 7 Kohlenstoffatomen im Ring und der Ring kann statt der jeweiligen Kohlenstoffatome zwei Sauerstoffatome oder zwei Sauerstoffatome und ein Stickstoffatom enthalten,
P für ein Wasserstoffatom steht,
mit einem Aminopyrimidin der Formel V umsetzt, 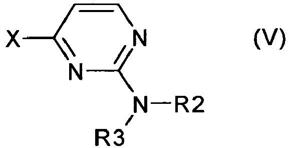 worin R2 und R3 die gleiche Bedeutung wie in Formel I haben und
X für
1) Halogen,
2) -O-SO₂-R2, oder
3) -O-C(O)-R2 steht,
oder
b) ein Boronoindol der Formel IV mit einem Pyrimidin der Formel VI, 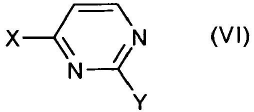
worin X die gleiche Bedeutung wie in Formel V hat und
Y für
1) Halogen,
2) -O-SO₂-R2, oder
3) -O-C(O)-R2 steht,
zu einer Verbindung der Formel VII umsetzt, 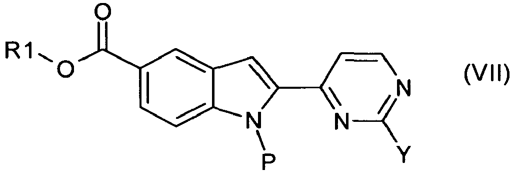 worin R1 und P die Bedeutung in der Verbindung der Formel IV haben und Y die gleiche Bedeutung wie in der Verbindung der Formel VI hat, und anschließend die Verbindung der Formel VII mit einem Amin der Formel VIII, 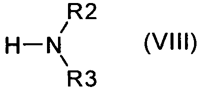 worin R2 und R3 die gleiche Bedeutung wie in Formel I haben,
zu einer Verbindung der Formel I umsetzt, und
c) die nach den Verfahren a) oder b) hergestellte Verbindung der Formel I entweder in freier Form isoliert oder im Falle des Vorliegens von sauren oder basischen Gruppen in physiologisch verträgliche Salze umwandelt.

2. Verfahren zur Gewinnung der Verbindung der Formel I nach Anspruch 1, wobei
R1 für
1) ein Wasserstoffatom,
2) -(C₁-C₆)-Alkyl,
3) Phenyl oder
4) -(C₃-C₆)-Cycloalkyl, steht,
R2 und R3 gleich oder verschieden sind und unabhängig voneinander für ein Wasserstoffatom oder -(C₁-C₄)-Alkyl, stehen,
**dadurch gekennzeichnet, dass** man ein Boronoindol der Formel IV,
worin R1 wie für Formel I definiert ist,
R4 und R5 gleich oder verschieden sind und unabhängig voneinander für -OH oder -(C₁-C₆)-Alkyl, stehen, oder
R4 und R5 bilden zusammen mit dem B-Atom, an das sie gebunden sind, einen Ring aus der Reihe Borolan, Borinan, Borepan, Borocan, [1,3,2]Dioxaborolan, [1,3,2]Dioxaborinan, [1,3,2]Dioxaborepan, [1,3,2]Dioxaborocan oder [1,3,6,2]Dioxazaborocan,
P für einWasserstoffatom steht,
mit einem Aminopyrimidin der Formel V umsetzt,
worin R2 und R3 gleich oder verschieden sind und unabhängig voneinander für ein Wasserstoffatom oder -(C₁-C₄)-Alkyl stehen, und
X für Cl, Br, I, -O-Tosylat, -O-Mesylat oder -O-Acetat steht.

3. Verfahren zur Gewinnung der Verbindung der Formel I nach Anspruch 1 oder 2, wobei
R1 für ein Wasserstoffatom oder Ethyl steht,
R2 und R3 gleich oder verschieden sind und unabhängig voneinander für ein Wasserstoffatom oder -(C₁-C₄)-Alkyl, stehen,
**dadurch gekennzeichnet, dass** man ein Boronoindol der Formel IV,
worin R1 wie für Formel I definiert ist,
R4 und R5 gleich sind und für -OH stehen, und
P für ein Wasserstoffatom steht,
mit einem Aminopyrimidin der Formel V umsetzt,
worin R2 und R3 gleich oder verschieden sind und unabhängig voneinander für ein Wasserstoffatom oder -(C₁-C₄)-Alkyl stehen, und
X für Cl, Br, I, -O-Tosylat, -O-Mesylat oder -O-Acetat steht.

4. Verfahren nach einem oder mehreren der Ansprüche 1 bis 3, **dadurch gekennzeichnet, dass** die Umsetzung der Boronoindole der Formel IV mit den Pyrimidinderivaten der Formel V in Gegenwart katalytischer Mengen an Palladium- oder Nickelverbindungen wie Pd(PPh₃)₄, Pd₂(dba)₃, Pd(OAc)₂ oder PdCl₂/TPPTS durchgeführt wird.

5. Verfahren nach Anspruch 4, **dadurch gekennzeichnet, dass** die Reaktionstemperatur von 40 °C bis 80 °C, bevorzugt von 60 °C bis 70 °C beträgt.

6. Verfahren nach Anspruch 4 oder 5, **dadurch gekennzeichnet, dass** das molare Verhältnis der Verbindung der Formel IV zur Verbindung der Formel V bei 1:1 bis 1:1,3 liegt.

7. Verfahren zur Gewinnung der Verbindung der Formel I nach Anspruch 1, wobei
R1 für
1) ein Wasserstoffatom,
2) -(C₁-C₆)-Alkyl,
3) Phenyl oder
4) -(C₃-C₆)-Cycloalkyl, steht,
R2 und R3 gleich oder verschieden sind und unabhängig voneinander für ein Wasserstoffatom oder -(C₁-C₄)-Alkyl, stehen,
**dadurch gekennzeichnet, dass** man ein Boronoindol der Formel IV,
worin R1 wie für Formel I definiert ist,
R4 und R5 gleich oder verschieden sind und unabhängig voneinander für -OH oder -(C₁-C₆)-Alkyl, stehen, oder
R4 und R5 bilden zusammen mit dem B-Atom, an das sie gebunden sind, einen Ring aus der Reihe Borolan, Borinan, Borepan, Borocan, [1,3,2]Dioxaborolan, [1,3,2]Dioxaborinan, [1,3,2]Dioxaborepan, [1,3,2]Dioxaborocan oder [1,3,6,2]Dioxazaborocan,
P für ein Wasserstoffatom steht,
mit einem Pyrimidin der Formel VI, worin
X für Cl, Br, I, -O-Tosylat, -O-Mesylat oder -O-Acetat steht, und
Y für Cl, Br, I, -O-Tosylat, -O-Mesylat oder -O-Acetat steht,
zu einer Verbindung der Formel VII umsetzt und anschließend die Verbindung der Formel VII mit einem Amin der Formel VIII, worin R2 und R3 gleich oder verschieden sind und unabhängig voneinander für ein Wasserstoffatom oder -(C₁-C₄)-Alkyl stehen, zu einer Verbindung der Formel I umsetzt.

8. Verfahren zur Gewinnung der Verbindung der Formel I nach Anspruch 7, wobei
R1 für ein Wasserstoffatom oder Ethyl steht,
R2 und R3 gleich oder verschieden sind und unabhängig voneinander für ein Wasserstoffatom oder -(C₁-C₄)-Alkyl, stehen,
**dadurch gekennzeichnet, dass** man ein Boronoindol der Formel IV,
worin R1 wie für Formel I definiert ist,
R4 und R5 gleich sind und für -OH stehen,
P für ein Wasserstoffatom steht,
mit einem Pyrimidin der Formel VI, worin
X für Cl, Br, I, -O-Tosylat, -O-Mesylat oder -O-Acetat steht, und
Y für Cl, Br, I, -O-Tosylat, -O-Mesylat oder -O-Acetat steht,
zu einer Verbindung der Formel VII umsetzt und anschließend die Verbindung der Formel VII mit einem Amin der Formel VIII, worin R2 und R3 gleich oder verschieden sind und unabhängig voneinander für ein Wasserstoffatom oder -(C₁-C₄)-Alkyl stehen, zu einer Verbindung der Formel I umsetzt.

9. Verbindung der Formel IV, 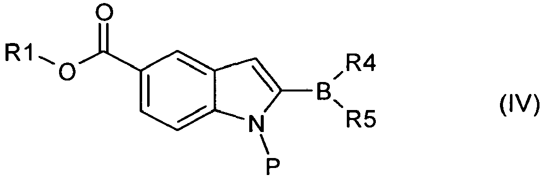 worin
R1 für
1) ein Wasserstoffatom,
2) -(C₁-C₁₂)-Alkyl,
3) -(C₆-C₁₄)-Aryl,
4) -(C₃-C₆)-Cycloalkyl oder
5) 4- bis 15-gliedriger Het-Ring, steht,
R4 und R5 gleich oder verschieden sind und unabhängig voneinander für
1) -OH,
2) -O-(C₁-C₁₂)-Alkyl,
3) -O-(C₆-C₁₄)-Aryl,
4) -O-(C₃-C₈)-Cycloalkyl,
5) -(C₁-C₁₂)-Alkyl oder
6) -O-Het, wobei Het ein 4- bis 15-gliedriger Het-Ring bedeutet, stehen, oder
R4 und R5 bilden zusammen mit dem B-Atom, an das sie gebunden sind, einen Ring mit 4, 5, 6 oder 7 Kohlenstoffatomen im Ring und der Ring kann statt der jeweiligen Kohlenstoffatome zwei Sauerstoffatome oder zwei Sauerstoffatome und ein Stickstoffatom enthalten,
P für ein Wasserstoffatom steht,
mit der Maßgabe, dass R4 und R5 nicht -OH oder -O-(C₁-C₁₂)-Alkyl sind, wenn R1 für -(C₁-C₁₂)-Alkyl steht.

10. Verbindung der Formel IV gemäß Anspruch 9, wobei
R1 für
1) ein Wasserstoffatom,
2) -(C₁-C₈)-Alkyl,
3) -(C₆-C₁₄)-Aryl, wobei Aryl ausgewählt ist aus der Reihe Phenyl, Naphthyl, Anthryl oder Fluorenyl,
4) -(C₃-C₆)-Cycloalkyl oder
5) 4- bis 15-gliedriger Het-Ring worin Het für einen Rest aus der Reihe Acridinyl, Azepinyl, Azetidinyl, Aziridinyl, Benzimidazalinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochro-manyl, Isoindazolyl, Isoindolinyl, Isoindolyl, Isochinolinyl, Benzimidazolyl, Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Oxothiolanyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Thiomorpholinyl, Thiophenyl, Triazinyl, 1,2,3-Triazolyl, 1,2,3-Triazolyl, 1,2,4-Triazolyl, 1,2,5-Triazolyl, 1,3,4-Triazolyl oder Xanthenyl, steht,
R4 und R5 gleich oder verschieden sind und unabhängig voneinander für
1) -OH,
2) -O-(C₁-C₁₂)-Alkyl,
3) -O-(C₆-C₁₄)-Aryl, worin Aryl wie oben definiert ist,
4) -O-(C₃-C₆)-Cycloalkyl,
5) -(C₁-C₆)-Alkyl oder
6) -O-Het, wobei Het ein 4- bis 15-gliedriger Het-Ring bedeutet und wie oben definiert ist, stehen, oder
R4 und R5 bilden zusammen mit dem B-Atom, an das sie gebunden sind, einen Ring aus der Reihe Borolan, Borinan, Borepan, Borocan, [1,3,2]Dioxaborolan, [1,3,2]Dioxaborinan, [1,3,2]Dioxaborepan, [1,3,2]Dioxaborocan oder [1,3,6,2]Dioxazaborocan, und
P für ein Wasserstoffatom steht.

11. Verfahren zur Herstellung der Verbindung der Formel IV gemäß Anspruch 9 oder 10, **dadurch gekennzeichnet, dass** man eine Verbindung der Formel XI 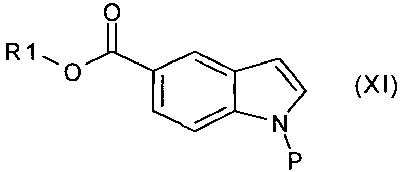 worin
R1 wie für die Verbindung der Formel IV definiert ist und
P für
1) ein Wasserstoffatom,
2) -C(O)-O-R6, worin R6 für
a) Wasserstoffatom,
b) -(C₁-C₁₂)-Alkyl,
c) -(C₆-C₁₄)-Aryl, wobei Aryl unsubstituiert oder ein-, zwei- oder dreifach durch -(C₁-C₆)-Alkyl substituiert ist,
d) -(C₃-C₈)-Cycloalkyl oder
e) 4- bis 15-gliedriger Het-Ring steht,
3) -SO₃-R6,
4) -O-SO₂-R6,
5) -Si-R6 oder
6) Benzyl steht,
mit Basen und anschließend mit dem Borsäureester B(R4)-R5, worin R4 und R5 wie für die Verbindung der Formel IV definiert sind, zu einer Verbindung der Formel IV umsetzt, wobei die gegebenenfalls vorhandene N-Schutzgruppe abgespalten wird.

## Claims

1. A process for obtaining the compound of the formula I 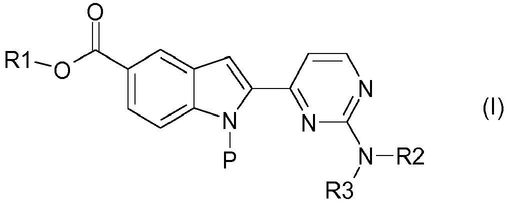 in which
R1 is
1) a hydrogen atom,
2) -(C₁-C₁₂)-alkyl,
3) -(C₆-C₁₄)-aryl,
4) -(C₃-C₈)-cycloalkyl or
5) a 4- to 15-membered Het ring,
R2 and R3 are the same or different and are each independently
1) a hydrogen atom,
2) (C₁-C₁₂)-alkyl,
3) -(C₆-C₁₄)-aryl where aryl is unsubstituted or mono-, di- or trisubstituted by -(C₁-₆)-alkyl,
4) -(C₃-C₈)-cycloalkyl or
5) a 4- to 15-membered Het ring,
P is a hydrogen atom,
which comprises
a) reacting a boronoindole of the formula IV 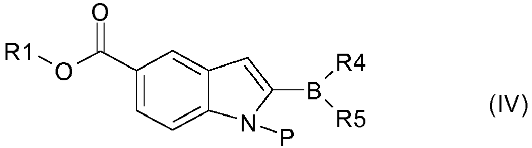
in which R1 is as defined in formula I,
R4 and R5 are the same or different and are each independently
1) -OH,
2) -O-(C₁-C₁₂)-alkyl,
3) -O-(C₆-C₁₄)-aryl,
4) -O-(C₃-₈)-cycloalkyl,
5) -(C₁-C₁₂)-alkyl or
6) -O-Het, where Het is a 4- to 15-membered Het ring,
or
R4 and R5, together with the boron atom to which they are bonded, form a ring having 4, 5, 6 or 7 carbon atoms in the ring and the ring may, instead of the particular carbon atoms, contain two oxygen atoms or two oxygen atoms and one nitrogen atom,
P is a hydrogen atom,
with an aminopyrimidine of the formula V 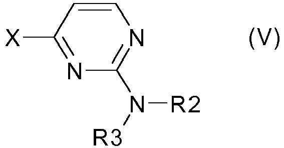 in which R2 and R3 are each as defined in formula I and
X is
1) halogen,
2) -O-SO₂-R2 or
3) -O-C(O)-R2,
or
b) reacting a boronoindole of the formula IV with a pyrimidine of the formula VI 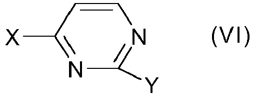 in which X is as defined in formula V and Y is
1) halogen,
2) -O-SO₂-R2, or
3) -O-C(O)-R2
to give a compound of the formula VII 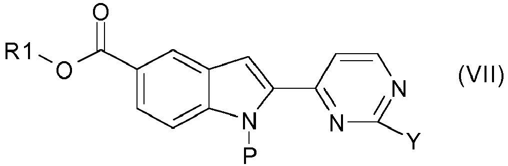 in which R1 and P are each as defined in formula IV and Y is as defined in the compound of the formula VI,
and then reacting the compound of the formula VII with an amine of the formula VIII 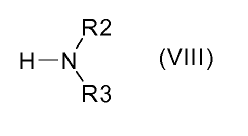 in which R2 and R3 are each as defined in formula I, and
c) either isolating the compound of the formula I prepared by processes a) or b) in free form or, in the case of the presence of acidic or basic groups, converting it to physiologically compatible salts.

2. The process for obtaining the compound of the formula I as claimed in claim 1 where
R1 is
1) a hydrogen atom,
2) -(C₁-C₆)-alkyl,
3) phenyl or
4) -(C₃-C₆)-cycloalkyl,
R2 and R3 are the same or different and are each independently a hydrogen atom or -(C₁-C₄)-alkyl,
wherein a boronoindole of the formula IV
in which R1 is as defined for formula I,
R4 and R5 are the same or different and are each independently -OH or -(C₁-C₆)-alkyl, or
R4 and R5, together with the boron atom to which they are bonded, form a ring from the group of borolane, borinane, borepane, borocane, [1,3,2]dioxaborolane, [1,3,2]dioxaborinane, [1,3,2]dioxaborepane, [1,3,2]dioxaborocane or [1,3,6,2]dioxazaborocane,
P is a hydrogen atom,
is reacted with an aminopyrimidine of the formula V
in which R2 and R3 are the same or different and are each independently a hydrogen atom or -(C₁-C₄)-alkyl, and
X is Cl, Br, I, -O-tosylate, -O-mesylate or -O-acetate.

3. The process for obtaining the compound of the formula I as claimed in claim 1 or 2 where
R1 is a hydrogen atom or ethyl,
R2 and R3 are the same or different and are each independently a hydrogen atom or -(C₁-C₄)-alkyl,
wherein a boronoindole of the formula IV
in which R1 is as defined for formula I,
R4 and R5 are the same and are each -OH, and
P is a hydrogen atom,
is reacted with an aminopyrimidine of the formula V
in which R2 and R3 are the same or different and are each independently a hydrogen atom or -(C₁-C₄)-alkyl, and
X is Cl, Br, I, -O-tosylate, -O-mesylate or -O-acetate.

4. The process as claimed in one or more of claims 1 to 3, wherein the reaction of the boronoindoles of the formula IV with the pyrimidine derivatives of the formula V is performed in the presence of catalytic amounts of palladium or nickel compounds such as Pd(PPh₃)₄, Pd₂(dba)₃, Pd(OAc)₂ or PdCl₂/TPPTS.

5. The process as claimed in claim 4, wherein the reaction temperature is from 40°C to 80°C, preferably from 60°C to 70°C.

6. The process as claimed in claim 4 or 5, wherein the molar ratio of the compound of the formula IV to the compound of the formula V is from 1:1 to 1:1.3.

7. The process for obtaining the compound of the formula I as claimed in claim 1 where
R1 is
1) a hydrogen atom,
2) -(C₁-C₆)-alkyl,
3) phenyl or
4) -(C₃-C₆)-cycloalkyl,
R2 and R3 are the same or different and are each independently a hydrogen atom or -(C₁-C₄)-alkyl,
wherein a boronoindole of the formula IV
in which R1 is as defined for formula I,
R4 and R5 are the same or different and are each independently -OH or -(C₁-C₆)-alkyl, or
R4 and R5, together with the boron atom to which they are bonded, form a ring from the group of borolane, borinane, borepane, borocane, [1,3,2]dioxaborolane, [1,3,2]dioxaborinane, [1,3,2]dioxaborepane, [1,3,2]dioxaborocane or [1,3,6,2]dioxazaborocane,
P is a hydrogen atom,
is reacted with a pyrimidine of the formula VI in which
X is Cl, Br, I, -O-tosylate, -O-mesylate or -O-acetate, and
Y is Cl, Br, I, -O-tosylate, -O-mesylate or O-acetate,
to give a compound of the formula VII and then the compound of the formula VII is reacted with an amine of the formula VIII in which R2 and R3 are the same or different and are each independently a hydrogen atom or -(C₁-C₄)-alkyl to give a compound of the formula I.

8. The process for obtaining the compound of the formula I as claimed in claim 7 where
R1 is a hydrogen atom or ethyl,
R2 and R3 are the same or different and are each independently a hydrogen atom or -(C₁-C₄)-alkyl,
wherein a boronoindole of the formula IV
in which R1 is as defined for formula I,
R4 and R5 are the same and are each -OH, and
P is a hydrogen atom,
is reacted with a pyrimidine of the formula VI in which
X is Cl, Br, I, -O-tosylate, -O-mesylate or -O-acetate, and
Y is Cl, Br, I, -O-tosylate, -O-mesylate or O-acetate,
to give a compound of the formula VII and then the compound of the formula VII is reacted with an amine of the formula VIII in which R2 and R3 are the same or different and are each independently a hydrogen atom or -(C₁-C₄)-alkyl to give a compound of the formula I.

9. A compound of the formula IV 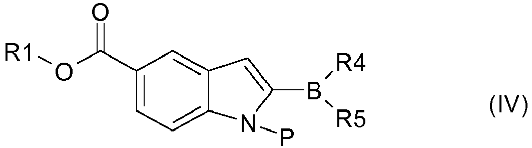 in which
R1 is
1) a hydrogen atom,
2) -(C₁-C₁₂)-alkyl,
3) -(C₆-C₁₄)-aryl,
4) -(C₃-C₆)-cycloalkyl or
5) a 4- to 15-membered Het ring,
R4 and R5 are the same or different and are each independently
1) -OH,
2) -O-(C₁-C₁₂)-alkyl,
3) -O-(C₆-C₁₄)-aryl,
4) -O-(C₃-₈)-cycloalkyl,
5) -(C₁-C₁₂)-alkyl or
6) -O-Het, where Het is a 4- to 15-membered Het ring,
or
R4 and R5, together with the boron atom to which they are bonded, form a ring having 4, 5, 6 or 7 carbon atoms in the ring and the ring may, instead of the particular carbon atoms, contain two oxygen atoms or two oxygen atoms and one nitrogen atom,
P is a hydrogen atom,
with the proviso that R4 and R5 are not -OH or -O-(C₁-C₁₂)-alkyl when R is -(C₁-C₁₂)-alkyl.

10. A compound of the formula IV as claimed in claim 9 where
R1 is
1) a hydrogen atom,
2) -(C₁-C-₈)-alkyl,
3) -(C₆-C₁₄)-aryl where aryl is selected from the group of phenyl, naphthyl, anthryl and fluorenyl,
4) -(C₃-C₆)-cycloalkyl or
5) a 4- to 15-membered Het ring, in which Het is a radical from the group of acridinyl, azepinyl, azetidinyl, aziridinyl, benzimidazalinyl, benzimidazolyl, benzofuranyl, benzothiofuranyl, benzothiophenyl, benzoxazolyl, benzothiazolyl, benzotriazolyl, benzo-tetrazolyl, benzoisoxazolyl, benzoisothiazolyl, carbazolyl, 4aH-carbazolyl, carbolinyl, quinazolinyl, quinolinyl, 4H-quinolizinyl, quinoxalinyl, quinuclidinyl, chromanyl, chromenyl, cinnolinyl, decahydroquinolinyl, dibenzofuranyl, dibenzothiophenyl, dihydrofuran[2,3-b]-tetrahydrofuranyl, dihydrofuranyl, dioxolyl, dioxanyl, 2H, 6H-1,5,2-dithiazinyl, furanyl, furazanyl, imidazolidinyl, imidazolinyl, imidazolyl, 1 H-indazolyl, indolinyl, indolizinyl, indolyl, 3H-indolyl, isobenzofuranyl, isochromanyl, isoindazolyl, isoindolinyl, isoindolyl, isoquinolinyl, benzoimidazolyl, isothiazolidinyl, 2-isothiazolinyl, isothiazolyl, isoxazolyl, isoxazolidinyl, 2-isoxazolinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolidinyl, oxothiolanyl, pyrimidinyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyroazolidinyl, pyrazolinyl, pyrazolyl, pyridazinyl, pyridooxazolyl, pyridoimidazolyl, pyridothiazolyl, pyridothiophenyl, pyridyl, pyrimidinyl, pyrrolidinyl, pyrrolinyl, 2H-pyrrolyl, pyrrolyl, tetrahydrofuranyl, tetrahydroisoquinolinyl, tetrahydroquinolinyl, tetrahydropyridinyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thieno-thiazolyl, thienooxazolyl, thienoimidazolyl, thiomorpholinyl, thiophenyl, triazinyl, 1,2,3-triazolyl, 1,2,3-triazolyl, 1,2,4-triazolyl, 1,2,5-triazolyl, 1,3,4-triazolyl or xanthenyl,
R4 and R5 are the same or different and are each independently
1) -OH,
2) -O-(C₁-C₁₂)-alkyl,
3) -O-(C₆-C₁₄)-aryl, where aryl is as defined above,
4) -O-(C₃-C₆)-cycloalkyl,
5) -(C₁-₆)-alkyl or
6) -O-Het, where Het is a 4- to 15-membered Het ring and is as defined above, or
R4 and R5, together with the boron atom to which they are bonded, form a ring from the group of borolane, borinane, borepane, borocane, [1,3,2]dioxaborolane, [1,3,2]dioxaborinane, [1,3,2]dioxaborepane, [1,3,2]dioxaborocane or [1,3,6,2]dioxazaborocane, and
P is a hydrogen atom.

11. A process for preparing the compound of the formula IV as claimed in claim 9 or 10, which comprises reacting a compound of the formula XI 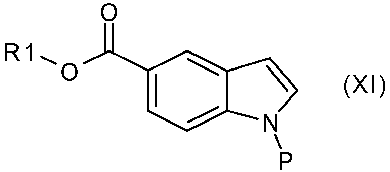
in which R1 is as defined for the compound of the formula IV, and P is
1) a hydrogen atom,
2) -C(O)-O-R6 in which R6 is
a) a hydrogen atom,
b) -(C₁-C₁₂)-alkyl,
c) -(C₆-₁₄)-aryl where aryl is unsubstituted or mono-, di- or trisubstituted by -(C₁-C₆)-alkyl,
d) -(C₃-C₈)-cycloalkyl or
e) a 4- to 15-membered Het ring,
3) -SO₃-R₆,
4) -O-SO₂-R₆,
5) -Si-R6 or
6) benzyl,
is reacted with bases and then with the boric ester B(R4)-R5 in which R4 and R5 are each as defined for the compound of the formula IV to give a compound of the formula IV and any nitrogen protecting group present is detached.

## Revendications

1. Procédé pour la production du composé de formule I, 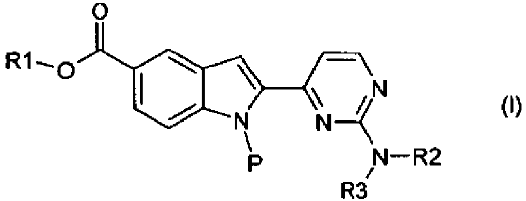 dans laquelle
R1 représente
1) un atome d'hydrogène,
2) -(C₁-C₁₂)-alkyle,
3) -(C₆-C₁₄)-aryle,
4) -(C₃-C₈)-cycloalkyle ou
5) hétérocycle de 4 à 15 chaînons,
R2 et R3 sont identiques ou différents et représentent, indépendamment l'un de l'autre
1) un atome d'hydrogène,
2) -(C₁-C₁₂)-alkyle,
3) -(C₆-C₁₄)-aryle, aryle étant non substitué ou monosubstitué, disubstitué ou trisubstitué par -(C₁-C₆)-alkyle,
4) -(C₃-C₈)-cycloalkyle ou
5) hétérocycle de 4 à 15 chaînons,
P représente un atome d'hydrogène,
**caractérisé en ce que**
a) on transforme un borono-indole de formule IV 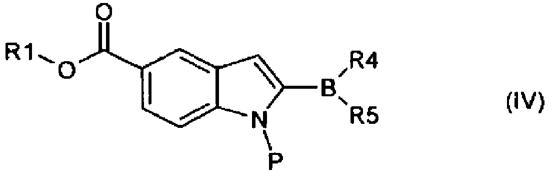 dans laquelle
R1 a la même signification que dans la formule I,
R4 et R5 sont identiques ou différents et représentent, indépendamment l'un de l'autre
1) -OH,
2) -O-(C₁-C₁₂)-alkyle,
3) -O-(C₆-C₁₄)-aryle,
4) -O-(C₃-C₈)-cycloalkyle,
5) -(C₁-C₁₂)-alkyle ou
6) -O-Het, Het signifiant un hétérocycle de 4 à 15 chaînons, ou
R4 et R5 forment, ensemble avec l'atome de B auquel ils sont liés, un cycle comprenant 4, 5, 6 ou 7 atomes de carbone dans le cycle et le cycle peut contenir, au lieu des atomes de carbone respectifs, deux atomes d'oxygène ou deux atomes d'oxygène et un atome d'azote,
P représente un atome d'hydrogène,
avec une aminopyrimidine de formule V, 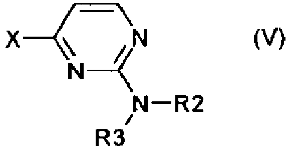
dans laquelle R2 et R3 ont la même signification que dans la formule I et
X représente
1) halogène,
2) -O-SO₂-R2, ou
3) -O-C(O)-R2,
ou
b) on transforme un borono-indole de formule IV avec une pyrimidine de formule VI, 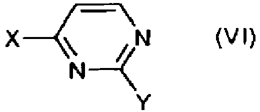 dans laquelle X a la même signification que dans la formule V et Y représente
1) halogène,
2) -O-SO₂-R2, ou
3) -O-C(O)-R2,
en un composé de formule VII 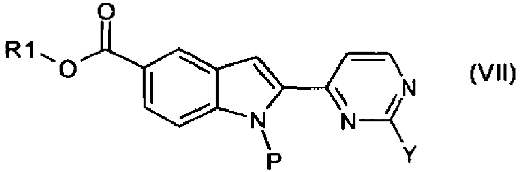 dans laquelle R1 et P ont la même signification que dans le composé de formule IV et Y a la même signification que dans le composé de formule VI et on transforme ensuite le composé de formule VII avec une amine de formule VIII, 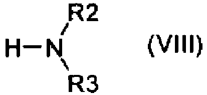 dans laquelle R2 et R3 ont la même signification que dans la formule I, en un composé de formule I, et
c) soit on isole le composé de formule I, préparé selon les procédés a) ou b) sous forme libre, soit, dans le cas de la présence de groupes acides ou basiques, il est converti en sels physiologiquement acceptables.

2. Procédé pour la production du composé de formule I selon la revendication 1,
R1 représentant
1) un atome d'hydrogène,
2) -(C₁-C₆)-alkyle,
3) phényle ou
4) -(C₃-C₆)-cycloalkyle,
R2 et R3 étant identiques ou différents et représentant, indépendamment l'un de l'autre, un atome d'hydrogène ou -(C₁-C₄)-alkyle,
**caractérisé en ce qu'**on transforme un borono-indole de formule IV, dans laquelle R1 est défini comme pour la formule I,
R4 et R5 sont identiques ou différents et représentent, indépendamment l'un de l'autre, -OH ou -(C₁-C₆)-alkyle, ou
R4 et R5 forment, ensemble avec l'atome de B auquel ils sont liés, un cycle de la série borolane, borinane, borépane, borocane, [1,3,2]dioxaborolane, [1,3,2]dioxaborinane, [1,3,2]dioxaborépane, [1,3,2]dioxaborocane ou [1,3,6,2]dioxazaborocane,
P représente un atome d'hydrogène,
avec une aminopyrimidine de formule V, dans laquelle R2 et R3 sont identiques ou différents et représentent, indépendamment l'un de l'autre, un atome d'hydrogène ou -(C₁-C₄)-alkyle, et
X représente Cl, Br, I, -O-tosylate, -O-mésylate ou -O-acétate.

3. Procédé pour la production du composé de formule I selon la revendication 1 ou 2,
R1 représentant un atome d'hydrogène ou éthyle,
R2 et R3
étant identiques ou différents et représentant, indépendamment l'un de l'autre, un atome d'hydrogène ou -(C₁-C₄)-alkyle,
**caractérisé en ce qu'**on transforme un borono-indole de formule IV,
dans laquelle R1 est défini comme pour la formule I,
R4 et R5 sont identiques et représentent -OH, et
P représente un atome d'hydrogène,
avec une aminopyrimidine de formule V, dans laquelle R2 et R3 sont identiques ou différents et représentent, indépendamment l'un de l'autre, un atome d'hydrogène ou -(C₁-C₄)-alkyle, et
X représente Cl, Br, I, -O-tosylate, -O-mésylate ou -O-acétate.

4. Procédé selon l'une ou plusieurs des revendications 1 à 3, **caractérisé en ce que** la transformation des borono-indoles de formule IV avec les dérivés de pyrimidine de formule V est réalisée en présence de quantités catalytiques de composés de palladium ou de nickel, tels que le Pd(PPh₃)₄, Pd₂(dba)₃, Pd(OAc)₂ ou PdCl₂/TPPTS.

5. Procédé selon la revendication 4, **caractérisé en ce que** la température de réaction est de 40°C à 80°C, de préférence de 60°C à 70°C.

6. Procédé selon la revendication 4 ou 5, **caractérisé en ce que** le rapport molaire du composé de formule IV au composé de formule V se situe à 1:1 jusqu'à 1:1,3.

7. Procédé pour la production du composé de formule I selon la revendication 1,
R représentant
1) un atome d'hydrogène,
2) -(C₁-C₆)-alkyle,
3) phényle ou
4) -(C₃-C₆)-cycloalkyle,
R2 et R3 étant identiques ou différents et représentant, indépendamment l'un de l'autre, un atome d'hydrogène ou -(C₁-C₄)-alkyle,
**caractérisé en ce qu'**on transforme un borono-indole de formule IV,
dans laquelle R1 est défini comme pour la formule I,
R4 et R5 sont identiques ou différents et représentent, indépendamment l'un de l'autre, -OH ou -(C₁-C₆)-alkyle, ou
R4 et R5 forment, ensemble avec l'atome de B auquel ils sont liés, un cycle de la série borolane, borinane, borépane, borocane, [1,3,2]dioxaborolane, [1,3,2]dioxaborinane, [1,3,2]dioxaborépane, [1,3,2]dioxaborocane ou [1,3,6,2]dioxazaborocane,
P représente un atome d'hydrogène,
avec une pyrimidine de formule VI, dans laquelle
X représente Cl, Br, I, -O-tosylate, -O-mésylate ou -O-acétate et
Y représente Cl, Br, I, -O-tosylate, -O-mésylate ou -O-acétate,
en un composé de formule VII et on transforme ensuite le composé de formule VII avec un amine de formule VIII, dans laquelle R2 et R3 sont identiques ou différents et représentent, indépendamment l'un de l'autre, un atome d'hydrogène ou -(C₁-C₄)-alkyle, en un composé de formule I.

8. Procédé pour la production du composé de formule I selon la revendication 7,
R1 représentant un atome d'hydrogène ou éthyle,
R2 et R3 étant identiques ou différents et représentant, indépendamment l'un de l'autre, un atome d'hydrogène ou -(C₁-C₄)-alkyle,
**caractérisé en ce qu'**on transforme un borono-indole de formule IV,
dans laquelle R1 est défini comme pour la formule I,
R4 et R5 sont identiques et représentent -OH,
P représente un atome d'hydrogène,
avec une pyrimidine de formule VI, dans laquelle
X représente Cl, Br, I, -O-tosylate, -O-mésylate ou -O-acétate et
Y représente Cl, Br, I, -O-tosylate, -O-mésylate ou -O-acétate,
en un composé de formule VII et on transforme ensuite le composé de formule VII avec un amine de formule VIII, dans laquelle R2 et R3 sont identiques ou différents et représentent, indépendamment l'un de l'autre, un atome d'hydrogène ou -(C₁-C₄)-alkyle, en un composé de formule I.

9. Composé de formule IV 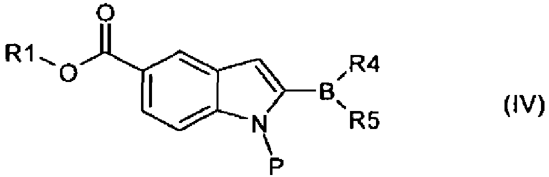 dans laquelle
R1 représente
1) un atome d'hydrogène,
2) -(C₁-C₁₂)-alkyle,
3) -(C₆-C₁₄)-aryle,
4) -(C₃-C₆)-cycloalkyle ou
5) hétérocycle de 4 à 15 chaînons,
R4 et R5 sont identiques ou différents et représentent, indépendamment l'un de l'autre
1) -OH,
2) -O-(C₁-C₁₂)-alkyle,
3) -O-(C₆-C₁₄)-aryle,
4) -O-(C₃-C₈)-cycloalkyle,
5) -(C₁-C₁₂)-alkyle ou
6) -O-Het, Het signifiant un hétérocycle de 4 à 15 chaînons,
ou
R4 et R5 forment, ensemble avec l'atome de B auquel ils sont liés, un cycle comprenant 4, 5, 6 ou 7 atomes de carbone dans le cycle et le cycle peut contenir, au lieu des atomes de carbone respectifs, deux atomes d'oxygène ou deux atomes d'oxygène et un atome d'azote,
P représente un atome d'hydrogène,
à condition que R4 et R5 ne représentent pas -OH ou -O-(C₁-C₁₂)-alkyle lorsque R1 représente -(C₁-C₁₂)-alkyle.

10. Composé de formule IV selon la revendication 9,
R1 représentant
1) un atome d'hydrogène,
2) -(C₁-C₈)-alkyle,
3) -(C₆-C₁₄)-aryle, aryle étant choisi dans la série phényle, naphtyle, anthryle ou fluorényle,
4) -(C₃-C₆)-cycloalkyle ou
5) un hétérocycle de 4 à 15 chaînons, dans lequel hétéro représente un radical de la série acridinyle, azépynyle, azétidinyle, aziridinyle, benzimidazalinyle, benzimidazolyle, benzofurannyle, benzothiofurannyle, benzothiophényle, benzoxazolyle, benzothiazolyle, benzotriazolyle, benzotétrazolyle, benzisoxazolyle, benzisothiazolyle, carbazolyle, 4aH-carbazolyle, carbolinyle, quinazolinyle, quinoléinyle, 4H-quinolézinyle, quinoxalinyle, quinuclidinyle, chromanyle, chroményle, cinnolinyle, décahydroquinoléinyle, dibenzofurannyle, dibenzothiophényle, dihydrofurann[2,3-b]-tétrahydrofurannyle, dihydrofurannyle, dioxolyle, dioxanyle, 2H,6H-1,5,2-dithiazinyle, furannyle, furazanyle, imidazolidinyle, imidazolinyle, imidazolyle, 1 H-indazolyle, indolinyle, indolizinyle, indolyle, 3H-indolyle, isobenzofurannyle, isochromanyle, iso-indazolyle, iso-indolinyle, iso-indolyle, isoquinoléinyle, benzimidazolyle, isothiazolidinyle, 2-isothiazolinyle, isothiazolyle, isoxazolyle, isoxazolidinyle, 2-isoxazolinyle, morpholinyle, naphtyridinyle, octahydro-isoquinoléinyle, oxadiazolyle, 1,2,3-oxadiazolyle, 1,2,4-oxadiazolyle, 1,2,5-oxadiazolyle, 1,3,4-oxadiazolyle, oxazolidinyle, oxazolyle, oxazolidinyle, oxothiolanyle, pyrimidinyle, phénanthridinyle, phénanthrolinyle, phénazinyle, phénothiazinyle, phénoxathiinyle, phénoxazinyle, phtalazinyle, pipérazinyle, pipéridinyle, ptéridinyle, purynyle, pyrannyle, pyrazinyle, pyroazolidinyle, pyrazolinyle, pyrazolyle, pyridazinyle, pyridooxazolyle, pyrido-imidazolyle, pyridothiazolyle, pyridothiophényle, pyridyle, pyrimidinyle, pyrrolidinyle, pyrrolinyle, 2H-pyrrolyle, pyrrolyle, tétrahydrofurannyle, tétrahydro-isoquinoléinyle, tétrahydroquinoléinyle, tétrahydropyridinyle, 6H-1,2,5-thiadazinyle, 1,2,3-thiadiazolyle, 1,2,4-thiadiazolyle, 1,2,5-thiadiazolyle, 1,3,4-thiadiazolyle, thianthrényle, thiazolyle, thiényle, thiénothiazolyle, thiénooxazolyle, thiéno-imidazolyle, thiomorpholinyle, thiophényle, triazinyle, 1,2,3-triazolyle, 1,2,3-triazolyle, 1,2,4-triazolyle, 1,2,5-triazolyle, 1,3,4-triazolyle ou xanthényle,
R4 et R5 étant identiques ou différents et représentant, indépendamment l'un de l'autre
1) -OH,
2) -O-(C₁-C₁₂)-alkyle,
3) -O-(C₆-C₁₄)-aryle, dans laquelle aryle est défini comme ci-dessus,
4) -O-(C₃-C₆)-cycloalkyle,
5) -(C₁-C₆)-alkyle ou
6) -O-Het, Het signifiant un hétérocycle de 4 à 15 chaînons, et étant défini comme ci-dessus,
R4 et R5 formant, ensemble avec l'atome de B auquel ils sont liés, un cycle de la série borolane, borinane, borépane, borocane, [1,3,2]dioxaborolane, [1,3,2]dioxaborinane, [1,3,2]dioxaborépane, [1,3,2]dioxaborocane ou [1,3,6,2]dioxazaborocane, et
P représentant un atome d'hydrogène.

11. Procédé pour la préparation du composé de formule IV selon la revendication 9 ou 10, **caractérisé en ce qu'**on transforme un composé de formule XI 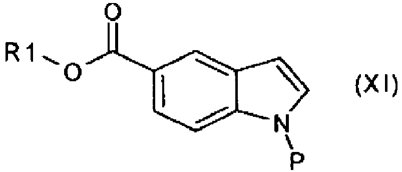 dans laquelle
R1 est défini comme pour le composé de formule IV et
P représente
1) un atome d'hydrogène,
2) -C(O)-O-R6, où R6 représente
a) un atome d'hydrogène,
b) -(C₁-C₁₂)-alkyle,
c) -(C₆-C₁₄)-aryle, aryle étant non substitué ou monosubstitué, disubstitué ou trisubstitué par (C₁-C₆)-alkyle,
d) -(C₃-C₈)-cycloalkyle ou
e) hétérocycle de 4 à 15 chaînons,
3) -SO₃-R6,
4) -O-SO₂-R6,
5) -Si-R6 ou
6) benzyle,
avec des bases et ensuite avec un ester de l'acide borique B(R4)-R5, dans lequel R4 et R5 sont définis comme pour le composé de formule IV, en un composé de formule IV, le groupe de protection de N le cas échéant présent étant dissocié.
